(19) Europäisches Patentamt
European Patent Office
Office européen des brevets

(11) **EP 3 035 042 A1**

(12) **EUROPÄISCHE PATENTANMELDUNG**

(43) Veröffentlichungstag:
**22.06.2016 Patentblatt 2016/25**

(51) Int Cl.:
***G01N 27/22*** *(2006.01)*

(21) Anmeldenummer: **14004272.2**

(22) Anmeldetag: **18.12.2014**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(71) Anmelder: **Airbus Defence and Space GmbH
85521 Ottobrunn (DE)**

(72) Erfinder: **Buchmann, Christopher
81377 München (DE)**

(54) **Sensoreinrichtung, Messanordnung und Materialparametererfassungs-Verfahren**

(57) Die Erfindung betrifft eine Sensoreinrichtung (28) zum Erfassen wenigstens eines Materialparameters eines Verbundwerkstoffs (26), der ein Matrixmaterial (24) aufweist, um Messdaten zu erhalten, mit einem zwischen einer Innenelektrode (30) und einer die Innenelektrode (30) im Querschnitt vollständig umgebenden Außenelektrode (34) vorgesehenen Probenraum (38) zum Aufnehmen des Matrixmaterials (24) und/oder des Verbundwerkstoffs (26).

Fig. 1

EP 3 035 042 A1

**Beschreibung**

Die Erfindung betrifft eine Sensoreinrichtung, eine Messanordnung und ein Materialparametererfassungsverfahren. Insbesondere betrifft die Erfindung eine Sensoreinrichtung, eine Messanordnung und ein Materialparametererfassungsverfahren zur Überwachung eines Herstellungsprozesses von Verbundwerkstoffen.

Aus Sernek und Kamke, Int. J. Adhes. Adhes., Vol. 27, no. 7, pp. 562-567, 2007 ist ein Verfahren und einen Kammsensor zur Übwerwachung eines Aushärtungsprozesses eines Phenolformaldehyd-Klebstoffes bekannt.

Kazilas und Partridge, Poymer, Vol. 46, no. 16, pp. 5868-5878 beschreiben ein Modell für einen Zusammenhang von Aushärtungstemperatur, Aushärtungsgrad und elektrischer Impedanz von Kammsensoren.

Hardis et al., Compos. Part Appl. Sci. Manuf., Vol 49, pp. 100-108, 2013 beschreiben einen Plattenkondensator zur Untersuchung der Aushärtungskinetik von Epoxidharz.

Die bekannten Sensoren erlauben eine punktuelle Messung des Verbundwerkstoffes. Zusätzliche Sensoren können die Messfläche vergrößern, sind jedoch mit hohem Aufwand und weiteren Kosten verbunden.

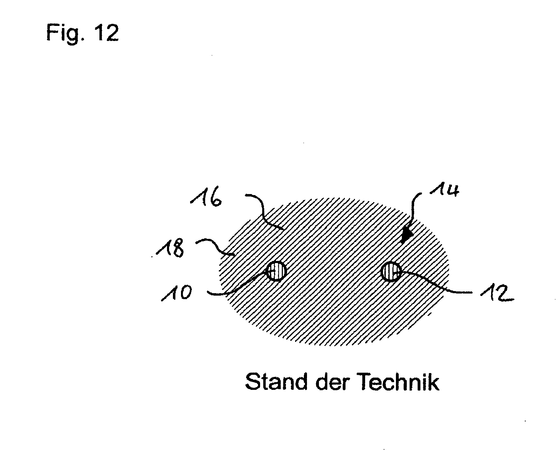

Eine Zweidrahtleitung, wie in Fig. 12 beispielhaft wiedergegeben, wurde in Dominauskas et al., Compos. Part Appl. Sci. Manuf., Vol. 34, pp. 67- 74, 2003 und Vol. 38, pp. 138-146, 2007 zur Überwachung eines Herstellungsverfahrens von Verbundwerkstoffen beschrieben.

Eine erste Leitung 10 wird an Erde angeschlossen, während eine zweite Leitung 12 mit einem Spannungsimpuls beaufschlagt wird. Die Leitungen 10, 12 werden in einem Dielektrikum 14 angeordnet. Das Dielektrikum 14 enthält ein Fasermaterial 16 und wird mit einem Harz 18 infusioniert. Durch Zeitbereichsreflektometrie lässt sich die Position der Fließfront des Harzes 18 und dessen Aushärtungsgrad bestimmen.

Derartige Sensoren umfassen zwei parallel angeordnete Leiterbahnen. Daher können elektrisch leitende Materialien (beispielsweise leitende Kohlenstofffasern, metallische Formwerkzeuge,...), die sich in der Nähe des Sensors befinden, das elektrische Feld der Sensoren zusätzlich beeinflussen und so eine präzise Messung zumindest erschweren. Mit der in Fig. 12 dargestellten Konfiguration können daher lediglich für nicht leitende Materialien, wie etwa Fasern, und nicht leitende Formwerkzeuge gute Ergebnisse erzielt werden.

Der Erfindung liegt die Aufgabe zugrunde, das Messen von Materialparametern, insbesondere hinsichtlich der Präzision, zu verbessern.

Als Lösung wird eine Sensoreinrichtung gemäß Anspruch 1 sowie eine Messanordnung und ein Materialparametererfassungsverfahren gemäß der weiteren unabhängigen Ansprüche vorgeschlagen. Vorteilhafte Weiterbildungen sind Gegenstand der abhängigen Ansprüche.

Gemäß einem Aspekt schafft die Erfindung eine Sensoreinrichtung zum Erfassen wenigstens eines Materialparameters eines Verbundwerkstoffs, der ein Matrixmaterial aufweist, um Messdaten zu erhalten, mit einem zwischen einer Innenelektrode und einer die Innenelektrode im Querschnitt vollständig umgebenden Außenelektrode vorgesehenen Probenraum zum Aufnehmen des Matrixmaterials und/oder des Verbundwerkstoffs.

Vorzugsweise umfasst die Sensoreinrichtung eine zweite Innenelektrode. Vorzugsweise ist die zweite Innenelektrode in dem Probenraum angeordnet und insbesondere im Querschnitt vollständig von der Außenelektrode umgeben.

Es ist bevorzugt, dass die Innenelektrode einen Draht aufweist. Es ist bevorzugt, dass die Innenelektrode eine Litze aufweist. Es ist bevorzugt, dass die Innenelektrode einen Flechtkern aufweist. Es ist bevorzugt, dass die Innenelektrode einen nach dem Aushärten des Verbundwerkstoffs aus diesem entfernbaren Abschnitt aufweist. Es ist bevorzugt, dass die Innenelektrode einen nach dem Aushärten des Verbundwerkstoffs aus diesem nicht-entfernbaren Abschnitt aufweist.

Es ist bevorzugt, dass die Außenelektrode, insbesondere im Querschnitt, ringförmig ausgebildet ist. Es ist bevorzugt, dass die Außenelektrode, insbesondere im Querschnitt, kreisringförmig ausgebildet ist. Es ist bevorzugt, dass die Außenelektrode, insbesondere im Querschnitt, rechteckig ausgebildet ist. Es ist bevorzugt, dass die Außenelektrode, insbesondere im Querschnitt, quadratisch ausgebildet ist. Es ist bevorzugt, dass die Außenelektrode abschnittsweise und/oder vollständig konzentrisch zu der Innenelektrode angeordnet ist.

Es ist bevorzugt, dass die Außenelektrode Öffnungen und/oder Poren aufweist. Es ist bevorzugt, dass die Außenelektrode ein poröses Material aufweist. Das poröse Material, die Öffnungen und/oder die Poren sind bevorzugt geeignet, ein Eindringen des Matrixmaterials in den Probenbraum zu gestatten. Beispiel für ein poröses Material ist etwa ein Geflecht oder Gelege aus Kupfer. Es ist bevorzugt, dass die Außenelektrode ein leitendes Material aufweist. Es ist bevorzugt, dass die Außenelektrode ein Geflecht aufweist. Es ist bevorzugt, dass die Außenelektrode ein Gelege aufweist. Es ist bevorzugt, dass die Außenelektrode eine Konsolidierungseinrichtung zum Aushärten des Verbundwerkstoffs aufweist.

Es ist bevorzugt, dass die Innenelektrode und/oder die Außenelektrode aus Metall und/oder Kupfer und/oder Stahl gefertigt sind.

Es ist bevorzugt, dass der Probenraum zum Aufnehmen eines Fasermaterials und/oder eines Prepregs und/oder eines Matrixmaterials und/oder des Verbundwerkstoffs ausgebildet ist.

Gemäß einem weiteren Aspekt schafft die Erfindung eine Messanordnung Messanordnung zum Ermitteln wenigstens eines Materialparameters eines Verbundwerkstoffs mit einer, insbesondere hierin beschriebenen, Sensoreinrichtung wobei der Probenraum ein Dielektrikum aufweist, das teilweise oder vollständig aus einem Fasermaterial und einem Matrixmaterial und/oder einem Prepreg und/oder dem Verbundwerkstoff gebildet ist und/oder das in der axialen Richtung der Sensoreinrichtung bewegbar ist.

Gemäß einem weiteren Aspekt schafft die Erfindung eine Messanordnung zum Ermitteln wenigstens eines Materialparameters eines Verbundwerkstoffs mit einer, insbesondere hierin beschriebenen, Materialparameterermittlungsvorrichtung, wobei der Probenraum ein Dielektrikum aufweist, das teilweise oder vollständig aus einem Fasermaterial und/oder einem Matrixmaterial und/oder einem Prepreg und/oder dem Verbundwerkstoff gebildet ist und/oder das in der axialen Richtung der Sensoreinrichtung bewegbar ist.

Es ist bevorzugt, dass die Innenelektrode einen Flechtkern aufweist. Es ist bevorzugt, dass die Außenelektrode eine Konsolidierungseinrichtung zum Aushärten des Verbundwerkstoffs aufweist.

Es ist bevorzugt, dass der Verbundwerkstoff aus einem Fasermaterial, insbesondere einem Fasergewebe und/oder Fasergelege, und einem Matrixmaterial und/oder einem Prepreg gebildet ist. Es ist bevorzugt, dass das Fasermaterial Glasfasern aufweist. Es ist bevorzugt, dass das Fasermaterial Kohlenstofffasern aufweist. Es ist bevorzugt, dass das Fasermaterial Aramidfasern aufweist.

Es ist bevorzugt, dass das Prepreg ein Fasergewebe aufweist. Es ist bevorzugt, dass das Prepreg ein Fasergelege aufweist. Es ist bevorzugt, dass das Prepreg Glasfasern aufweist. Es ist bevorzugt, dass das Prepreg Kohlenstofffasern aufweist. Es ist bevorzugt, dass das Prepreg Aramidfasern aufweist.

Es ist bevorzugt, dass das Matrixmaterial ein Harz enthält. Es ist bevorzugt, dass das Matrixmaterial ein Kunstharz enthält.

Gemäß einem weiteren Aspekt schafft die Erfindung ein Materialparametererfassungsverfahren zum Erfassen wenigstens eines Materialparameters eines Verbundwerkstoffs, mit den Schritten:

1 Anordnen des Verbundwerkstoffs in einem zwischen einer Innenelektrode und einer die Innenelektrode, insbesondere im Querschnitt vorzugsweise vollständig, umgebenden Außenelektrode vorgesehenen Probenraum, der bevorzugt zum Aufnehmen des Verbundwerkstoffs ausgebildet ist;

2 Einspeisen eines Detektionsspannungsimpulses mit einer Detektionsspannungsimpulsamplitude in die Innenelektrode;

4 Erfassen einer Reflexionsspannungsimpulslaufzeit und/oder einer Reflexionsspannungsimpulsamplitude eines aus dem Detektionsspannungsimpuls hervorgegangenen Reflexionsspannungsimpulses;

5 Ermitteln des Materialparameters aus der Reflexionsspannungsimpulslaufzeit und/oder der Reflexionsspannungsimpulsamplitude.

Vorzugsweise umfasst das Verfahren den Schritt:

3 Einspeisen eines zweiten Detektionsspannungsimpulses mit einer zweiten Detektionsspannungsimpulsamplitude in die Außenelektrode.

Vorzugsweise umfasst das Verfahren den Schritt:

6 Anschließen der Außenelektrode an Erde.

Vorzugsweise wird der Schritt 2 simultan/gleichzeitig mit Schritt 3 durchgeführt.

Es ist bevorzugt, dass die zweite Detektionsspannungsimpulsamplitude betragsmäßig gleich der ersten Detektionsspannungsimpulsamplitude ist.

Es ist bevorzugt, dass die zweite Detektionsspannungsimpulsamplitude das umgekehrte Vorzeichen der ersten Detektionsspannungsimpulsamplitude aufweist.

Es ist bevorzugt, dass Schritt 1 das Bewegen des Verbundwerkstoffs durch den Probenraum, insbesondere entlang der axialen Richtung des Probenraums, enthält.

Es ist bevorzugt, dass Schritt 1 das Relativbewegen des Verbundwerkstoffs relativ zu der Innenelektrode, insbesondere entlang der axialen Richtung der Innenelektrode enthält.

Es ist bevorzugt, dass Schritt 1 das Relativbewegen des Verbundwerkstoffs relativ zu der Außenelektrode, insbesondere entlang der axialen Richtung der Außenelektrode enthält.

Vorzugsweise wird Verfahren durch Verwenden einer hierin beschriebenen Sensoreinrichtung durchgeführt.

Vorzugsweise wird Verfahren durch Verwenden einer hierin beschriebenen Messanordnung durchgeführt.

Vorzugsweise wird Verfahren durch Verwenden einer hierin beschriebenen Materialparametererfassungsvorrichtung durchgeführt.

Gemäß einem weiteren Aspekt schafft die Erfindung eine Materialparameterermittlungsvorrichtung zum Ermitteln wenigstens eines Materialparameters eines Verbundwerkstoffs, mit einer, insbesondere hierin beschriebenen, Sensoreinrichtung, die zum Erfassen von physikalischen Parametern ausgebildet ist und die einen zwischen einer Innenelektrode und einer die Innenelektrode im Querschnitt vollständig umgebenden Außenelektrode vorgesehenen Probenraum zum Aufnehmen des Verbundwerkstoffs aufweist, und mit einer Steuereinrichtung, die zum Steuern der Sensoreinrichtung ausgebildet ist.

Es ist bevorzugt, dass die Steuereinrichtung ein Spannungsimpulserzeugungsmodul aufweist, das zum Erzeugen und Einspeisen wenigstens eines Spannungsimpulses mit einer vorbestimmen Spannungsimpulsamplitude ausgebildet ist.

Es ist bevorzugt, dass die Steuereinrichtung ein Spannungsimpulserfassungsmodul aufweist, das zum Erfassen einer Spannungsimpulsamplitude eines Spannungsimpulses ausgebildet ist.

Es ist bevorzugt, dass die Steuereinrichtung ein Spannungsimpulslaufzeiterfassungsmodul aufweist, das zum Erfassen einer Spannungsimpulslaufzeit ausgebildet ist.

Es ist bevorzugt, dass die Steuereinrichtung ein Auswertemodul aufweist, das zum Ermitteln eines Materialparameters des Verbundwerkstoffs aus einer Spannungsimpulsamplitude und/oder einer Spannungsimpulslaufzeit ausgebildet ist.

Es ist bevorzugt, dass das Spannungsimpulserzeugungsmodul ausgebildet ist, die Innenelektrode mit einem ersten Detektionsspannungsimpuls mit einer ersten Detektionsspannungsimpulsamplitude zu beaufschlagen.

Es ist bevorzugt, dass das Spannungsimpulserzeugungsmodul ausgebildet ist, die Außenelektrode mit einem zweiten Detektionsspannungsimpuls mit einer zweiten Detektionsspannungsimpulsamplitude zu beaufschlagen.

Es ist bevorzugt, dass das Spannungsimpulserfassungsmodul zum Erfassen einer Reflexionsspannungsimpulsamplitude eines aus einem Detektionsspannungsimpuls hervorgegangenen Reflexionsspannungsimpulses ausgebildet ist.

Es ist bevorzugt, dass das Spannungsimpulslaufzeiterfassungsmodul zum Erfassen einer Reflexionsspannungsimpulslaufzeit eines aus einem Detektionsspannungsimpuls hervorgegangenen Reflexionsspannungsimpulses ausgebildet ist.

Es ist bevorzugt, dass das Auswertemodul zum Ermitteln einer dielektrischen Permeabilität ausgebildet ist

Es ist bevorzugt, dass das Auswertemodul zum Ermitteln einer Position eines in dem Probenraum angeordneten Dielektrikums ausgebildet ist.

Vorzugsweise umfasst die Materialparametererfassungsvorrichtung eine Relativbewegungseinrichtung, die zum relativen Bewegen des Verbundwerkstoffs relativ zu der Innenelektrode und/oder zu der Außenelektrode durch den Probenraum ausgebildet ist.

Mit den hierin beschriebenen Vorrichtungen und Verfahren können insbesondere die nachfolgend erläuterten Vorteile erreicht werden.

Dielektrische Sensoreinrichtungen stellen eine wirkungsvolle Methode zur Überwachung der Fließfront und des Aushärtegrades bei Infusionsprozessen zum Herstellen von Verbundbauteilen, beispielsweise Faserverbundbauteilen, dar. Zum Überwachen größerer Flächen eignen sich vor allem längliche Sensoren, die etwa über Zeitbereichsreflektometrie ausgewertet werden können. Zeitbereichsreflektometrie wird auch Time-Domain-Reflectometry oder kurz TDR genannt.

Durch die Abschirmung der Innenelektrode durch die Außenelektrode entsteht ein Probenraum, der gegenüber Störfeldern aus der Umgebung abgeschirmt ist. Dennoch wird der Probenraum durch den Verbundwerkstoff, insbesondere das Fasermaterial und das Matrixmaterial, etwa Harz, beeinflusst und somit eine Messung ermöglicht. Beim Anlegen einer Spannung zwischen der Innenelektrode und der Außenelektrode bildet sich ein elektrisches Feld aus, das gegenüber der Umgebung geschirmt ist. Die dielektrischen Eigenschaften des isolierenden Mediums (Dielektrikum) zwischen Innenelektrode und Außenelektrode bestimmen die Eigenschaften des Sensors. Beispielsweise kann der Wellenwiderstand der Leitung mit einer geeigneten Messmethode, wie etwa TDR, gemessen werden.

Der Sensor kann als Dielektrikum zwischen der Innenelektrode und der Außenelektrode und auch zur Isolierung des gesamten Sensors ein poröses Material verwenden, z.B. ein Geflecht aus Glasfasern. Denkbar ist auch die Verwendung eines Prepregs. Der Außenleiter kann ebenfalls aus einem porösen und leitenden Material bestehen, z.B. einem Kupfergeflecht. Ein flüssiges Medium, etwa Harz während des Infusionierens, kann so das Dielektrikum tränken. Durch die sich verändernden Eigenschaften des Dielektrikums können beispielsweise die Position der Fließfront des Harzes oder eine Veränderung der dielektrischen Permittivität während der Aushärtung des Harzes ermittelt werden.

Durch die Schirmung des elektrischen Feldes ist der Sensor vergleichsweise unempfindlich gegenüber äußeren Einflüssen, wie etwa metallischen Werkzeugen oder leitenden Fasern. Somit kann die Präzision und/oder Qualität der Messung, insbesondere verglichen mit Zweidraht- oder Kammsensoren, verbessert werden.

Die Messung kann "single-ended" erfolgen, d.h. lediglich ein Leiter des Sensors wird mit einer Spannung beaufschlagt, während der andere Leiter als Erde dient. Zur weiteren Verbesserung des Signal-zu-Rausch-Abstandes kann die sogenannte differentielle TDR verwendet werden. Dabei werden beide Leiter des Sensors mit einem gegenpoligen Signal beaufschlagt. Bei gleichbleibender differentieller Signalamplitude können so Reflexionen an Störstellen entlang des Sensors reduziert und somit die Messqualität weiter verbessert werden.

Durch die Kombination einer geschirmten Elektrodengeometrie mit Innenelektrode und Außenelektrode sowie eines differentiellen TDR-Verfahrens kann die Fließfront eines infusionierten Harzes und/oder dessen Aushärtegrad genauer gemessen werden.

Durch die Verwendung differentieller TDR kann die Signalqualität, d.h. der Signal-zu-Rausch-Abstand, gesteigert werden. Zum Messen wird beispielsweise ein differentielles TDR-Messgerät verwendet, das über zwei Koaxialkabel mit dem Sensor verbunden wird. Die Verbindung ist derart aufgebaut, dass die Außenelektroden der Koaxialkabel kurz vor der Verbindung mit dem Sensor zusammengeführt werden. Damit ist die Verbindung zwischen Messgerät und Sensor nahezu vollständig geschirmt. Bereits ein einfacher hochtemperaturbeständiger Thermoelementstecker erzielt verbesserte Ergebnisse.

Die beschriebenen Vorrichtungen und Verfahren können insbesondere bei der Herstellung von Verbundwerkstoffen, insbesondere Faserverbundwerkstoffen, verwendet werden. Die Materialeigenschaften von Verbundwerkstoffen, wie beispielsweise Härte, Steifigkeit und Bruchfestigkeit, werden entscheidend bei der Herstellung bestimmt. Faserverbundwerkstoffe können allgemein aus einem Fasermaterial und einem Matrixmaterial hergestellt werden. Denkbar ist auch die Herstellung aus einem Prepreg. Als Fasermaterial werden beispielsweise Kohlenstofffasergewebe, Glasfasergewebe und dergleichen verwendet. Das Fasermaterial wird in einem sogenannten Infusionsverfahren mit dem Matrixmaterial verbunden. Dabei wird das Matrixmaterial, meist ein Kunstharz, als Bindemittel in das Fasermaterial eingespritzt. Das mit Matrixmaterial versehene Fasermaterial wird auch als Prepreg bezeichnet. Das Matrixmaterial wird anschließend ausgehärtet. Störstellen in dem Fasermaterial oder dem Matrixmaterial, wie beispielsweise Fehlstellen oder Luftblasen, beeinträchtigen die Stabilität des Verbundwerkstoffs. Sie sind daher möglichst zu vermeiden.

Mit den hierin beschriebenen Vorrichtungen und Verfahren können sowohl Fasermaterialien als auch Prepregs bereits vor dem Aushärten des Faserverbundbauteiles auf eventuelle Unregelmäßigkeiten zerstörungsfrei untersucht werden. Die Fehler können dann bereits vor dem Aushärten des Matrixmaterials ausgebessert werden.

Besonders vorteilhaft kann beim Flechten länglicher Faserverbundbauteile die Erfindung zur Überwachung des Herstellungsprozesses genutzt und in diesen integriert werden. Beispielsweise kann der Flechtkern für das Faserverbundbauteil als Innenelektrode fungieren. Als Außenelektrode kann ein einfacher Ring vorgesehen sein. Das geflochtene Fasermaterial kann demnach unmittelbar nach dem Flechten auf Fehler untersucht werden. Wird anschließend das Fasermaterial mit dem Matrixmaterial getränkt, kann das noch nicht ausgehärtete Prepreg erneut auf Fehler untersucht werden ohne die Produktionsstraße zu unterbrechen. Genügt das Faserverbundbauteil bis dato den Qualitätserfordernissen, so wird das Matrixmaterial in einer Konsolidierungseinrichtung ausgehärtet. Der Aushärtegrad des Matrixmaterials kann mit den hierin beschriebenen Vorrichtungen und Verfahren sowohl während des Aushärtens als auch in einer Abschlusskontrolle erfasst werden. Somit ist eine nahezu lückenlose Überwachung eines Herstellungsprozesses von Faserverbundbauteilen möglich.

Es sollte beachtet werden, dass die Nummerierung der Verfahrensschritte lediglich der Bezeichnung dient und keine Angabe für eine bestimmte Reihenfolge der Verfahrensschritte darstellt. Insbesondere kann die Reihenfolge, wo möglich, geändert werden. Verfahrensschritte können auch zeitgleich ausgeführt werden.

Ausführungsbeispiele der Erfindung wird nachfolgend anhand der beigefügten Zeichnung näher erläutert. Darin zeigt:

| Fig. 1 | eine schematische Ansicht eines Ausführungsbeispiel einer Materialparametererfassungsvorrichtung; |
| Fig. 2 | eine schematische Ansicht eines weiteren Ausführungsbeispiels einer Materialparametererfassungsvorrichtung; |
| Fig. 3 | eine Perspektivansicht eines Ausführungsbeispiels einer Sensoreinrichtung; |
| Fig. 4 | einen Querschnitt durch das Ausführungsbeispiel aus Fig. 3; |
| Fig. 5 | einen Querschnitt durch ein weiteres Ausführungsbeispiel einer Sensoreinrichtung; |
| Fig. 6 | eine Perspektivansicht eines weiteren Ausführungsbeispiels einer Sensoreinrichtung; |
| Fig. 7 | eine schematische Ansicht eines Ausführungsbeispiels einer Verbindungsanordnung für eine Sensoreinrichtung; |
| Fig. 8 | ein Spannungs-Zeit-Diagramm einer single-ended und einer differentiellen TDR-Messung; |
| Fig. 9 | ein weiteres Spannungs-Zeit-Diagramm einer differentiellen TDR-Messung; |
| Fig. 10A bis Fig. 10C | eine schematische Darstellung einer single-ended TDR-Messung; |
| Fig. 11A bis Fig. 11C | eine schematische Darstellung einer differentiellen TDR-Messung; und |
| Fig. 12 | einen schematischen Querschnitt durch ein Ausführungsbeispiel einer Sensoreinrichtung gemäß dem Stand der Technik. |

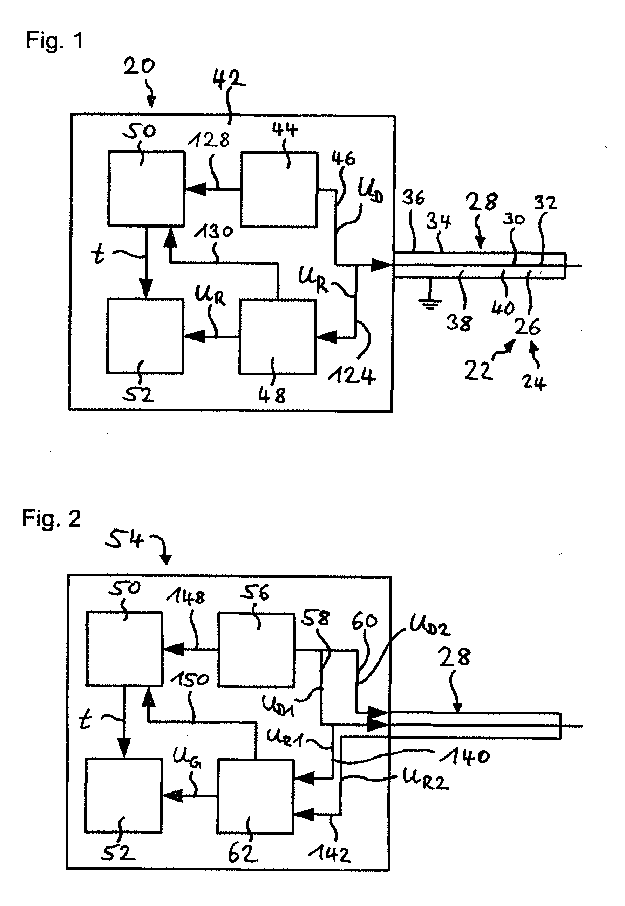

Fig. 1 zeigt schematisch ein Ausführungsbeispiel einer Materialparameterermittlungsvorrichtung 20.

Die Materialparameterermittlungsvorrichtung 20 ist ausgebildet, einen Materialparameter beispielsweise eines aus einem Fasermaterial 22 und einem Matrixmaterial 24 gebildeten Verbundwerkstoffes 26 zu ermitteln. Materialparameter sind insbesondere Fehlstellen in dem Verbundwerkstoff 26, der Aushärtungsgrad des Matrixmaterials 24 und die Verteilung des Matrixmaterials 24 in dem Verbundwerkstoff 26.

Die Materialparameterermittlungsvorrichtung 20 umfasst eine Sensoreinrichtung 28. Die Sensoreinrichtung 28 erstreckt sich beispielsweise in einer axialen Richtung.

Die Sensoreinrichtung 28 weist eine Innenelektrode 30 (Beispiel für eine erste Elektrode) auf. Die Innenelektrode 30 ist beispielsweise aus einem elektrisch leitenden Draht 32 gebildet.

Die Sensoreinrichtung 28 weist zudem eine Außenelektrode 34 (Beispiel für eine zweite Elektrode) auf. Die Außenelektrode 34 ist aus einem elektrisch leitenden Material, wie beispielsweise einem Geflecht 36 aus Kupfer, gebildet. Die Außenelektrode 34 ist beispielsweise kreisringförmig ausgebildet und umgibt im Querschnitt betrachtet die Innenelektrode 30 vollständig.

Die Sensoreinrichtung 28 weist ferner einen Probenraum 38 auf, der zwischen der Innenelektrode 30 und der Außenelektrode 34 angeordnet ist. Der Probenraum 38 wird von der Innenelektrode 30 und der Außenelektrode 34 begrenzt und ist ausgebildet, den Verbundwerkstoff 26 aufzunehmen. Der in dem Probenraum 38 aufgenommene Verbundwerkstoff 26 ist ein Beispiel für ein Dielektrikum 40.

Die Materialparameterermittlungsvorrichtung 20 umfasst zudem eine Steuereinrichtung 42. Die Steuereinrichtung 42 ist ausgebildet, die Sensoreinrichtung 28 zu steuern. Ferner ist die Steuereinrichtung 42 dazu ausgebildet, einen Materialparameter des Verbundwerkstoffs 26 zu ermitteln.

Die Steuereinrichtung 42 umfasst ein Spannungsimpulserzeugungsmodul 44. Das Spannungsimpulserzeugungsmodul 44 ist ausgebildet, einen Detektionsspannungsimpuls 46 mit einer Detektionsspannungsimpulsamplitude $U_D$ zu erzeugen. Ferner ist das Spannungsimpulserzeugungsmodul 44 dazu ausgebildet, die Sensoreinrichtung 28, insbesondere die Innenelektrode 30, mit dem Detektionsspannungsimpuls 46 zu beaufschlagen. Der Detektionsspannungsimpuls 46 kann beispielsweise eine rechteckige Impulsform aufweisen. Grundsätzlich sind auch andere Impulsformen denkbar.

Die Steuereinrichtung 42 umfasst ferner ein Spannungsimpulserfassungsmodul 48. Das Spannungsimpulserfassungsmodul 48 ist ausgebildet, eine Spannungsimpulsamplitude eines Spannungsimpulses zu erfassen.

Die Steuereinrichtung 42 umfasst weiter ein Spannungsimpulslaufzeiterfassungsmodul 50. Das Spannungsimpulslaufzeiterfassungsmodul 50 ist ausgebildet, eine Spannungsimpulslaufzeit eines Spannungsimpulses zu erfassen, wie noch beschrieben wird.

Die Steuereinrichtung 42 umfasst zudem ein Auswertemodul 52. Das Auswertemodul 52 ist ausgebildet, einen Materialparameter des Verbundwerkstoffes 26 aus einer Spannungsimpulslaufzeit und/oder einer Spannungsimpulsamplitude zu ermitteln.

Das Spannungsimpulserzeugungsmodul 44 ist mit der Innenelektrode 30 und mit dem Spannungsimpulslaufzeiterfassungsmodul 50 verbunden. Das Spannungsimpulserfassungsmodul 48 ist ebenfalls mit der Innenelektrode 30 und mit dem Spannungsimpulslaufzeiterfassungsmodul 50 verbunden. Zudem ist das Spannungsimpulserfassungsmodul 48 mit dem Auswertemodul 52 verbunden. Das Spannungsimpulslaufzeiterfassungsmodul 50 ist ebenso mit dem Auswertemodul 52 verbunden.

Es wird nachfolgend auf Fig. 2 Bezug genommen, die ein weiteres Ausführungsbeispiel einer Materialparameterermittlungsvorrichtung 54 zeigt.

Die Materialparameterermittlungsvorrichtung 54 ist ausgebildet, einen Materialparameter des Verbundwerkstoffs 26 zu ermitteln.

Die Materialparameterermittlungsvorrichtung 54 weist beispielsweise die Sensoreinrichtung 28 auf.

Die Materialparameterermittlungsvorrichtung 54 umfasst ferner ein Spannungsimpulserzeugungsmodul 56, das ausgebildet ist, einen ersten Detektionsspannungsimpuls 58 mit einer ersten Detektionsspannungsimpulsamplitude $U_{D1}$ und einen zweiten Detektionsspannungsimpuls 60 mit einer zweiten Detektionsspannungsimpulsamplitude $U_{D2}$ zu erzeugen. Das Spannungsimpulserzeugungsmodul 56 ist ferner ausgebildet, den ersten Detektionsspannungsimpuls 58 in die Innenelektrode 30 und den zweiten Detektionsspannungsimpuls 60 in die Außenelektrode 34 einzuspeisen.

Ferner umfasst die Materialparameterermittlungsvorrichtung 54 ein Spannungsimpulserfassungsmodul 62, das ausgebildet ist, aus zwei eingehenden Spannungsimpulsen mit jeweils einer Spannungsimpulsamplitude, insbesondere durch Differenzbildung, eine Spannungsimpulsamplitude zu ermitteln, wie noch beschrieben wird.

Im Übrigen umfasst die Materialparameterermittlungsvorrichtung 54 das Spannungsimpulslaufzeiterfassungsmodul 50 und das Auswertemodul 52.

Ähnlich wie bei dem vorigen Ausführungsbeispiel ist das Spannungsimpulserzeugungsmodul 56 mit dem Spannungsimpulslaufzeiterfassungsmodul 50 verbunden. Ferner ist das Spannungsimpulserfassungsmodul 62 mit dem Spannungsimpulslaufzeiterfassungsmodul 50 und dem Auswertemodul 52 verbunden. Wie zuvor ist das Spannungsimpulslaufzeiterfassungsmodul 50 ebenso mit dem Auswertemodul 52 verbunden.

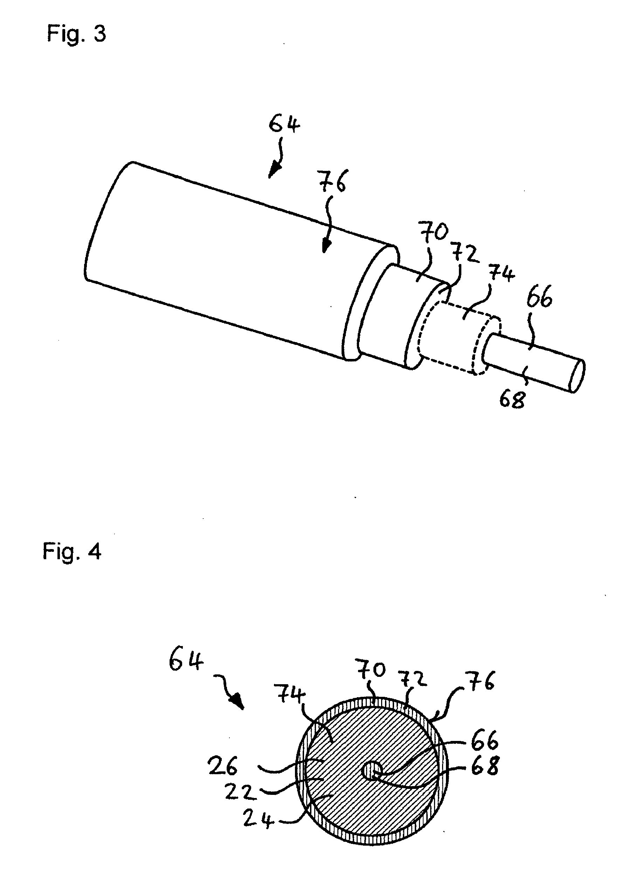

Es wird nachfolgend auf die Fig. 3 und 4 Bezug genommen, die ein Ausführungsbeispiel einer Sensoreinrichtung 64 zeigen. Die Sensoreinrichtung 64 erstreckt sich in einer axialen Richtung.

Die Sensoreinrichtung 64 umfasst eine Innenelektrode 66 (Beispiel für eine erste Elektrode), die beispielsweise eine Litze 68 aufweist. Die Innenelektrode 66 ist aus einem leitenden Material, wie etwa Kupfer, gefertigt.

Die Sensoreinrichtung 64 weist zudem eine Außenelektrode 70 (Beispiel für eine zweite Elektrode) auf. Die Außenelektrode 70 kann ein Geflecht 72 aufweisen. Die Außenelektrode 70 ist aus einem leitenden Material, wie etwa Kupfer, gefertigt. Die Außenelektrode 70 ist im Querschnitt kreisringförmig ausgebildet. Im vorliegenden Beispiel ist die Außenelektrode 70 konzentrisch zu der Innenelektrode 66 angeordnet. Die Außenelektrode 70 kann grundsätzlich auch nicht-konzentrisch angeordnet sein.

Die Sensoreinrichtung 64 weist einen Probenraum 74 auf, der zwischen der Innenelektrode 66 und der Außenelektrode 70 angeordnet ist. Der Probenraum 74 wird durch die Innenelektrode 66 und die Außenelektrode 70 begrenzt. Der Probenraum 74 ist ausgebildet, den Verbundwerkstoff 26 aufzunehmen.

Im vorliegenden Beispiel umfasst die Sensoreinrichtung 64 eine Isolierhülle 76. Die Isolierhülle 76 ist auf der äußeren Umfangsfläche der Außenelektrode 70 vorgesehen. Die Isolierhülle 76 kann beispielsweise einen Isolierlack oder einen isolierenden Gummi aufweisen.

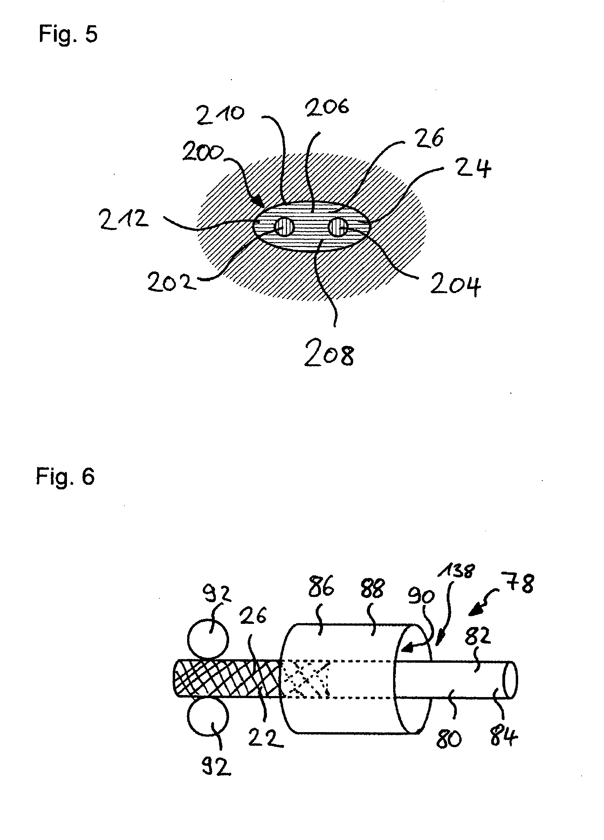

Fig. 5 zeigt ein Ausführungsbeispiel einer Sensoreinrichtung 200.

Die Sensoreinrichtung 200 umfasst eine erste Innenelektrode 202 (Beispiel für eine erste Elektrode) und eine zweite Innenelektrode 204 (Beispiel für eine zweite Elektrode). Die erste Innenelektrode 202 und die zweite Innenelektrode 204 sind im Wesentlichen parallel verlaufend zueinander angeordnet und voneinander durch ein Dielektrikum 206 beabstandet. Das Dielektrikum 206 kann den Verbundwerkstoff 26 und/oder das Fasermaterial 24 aufweisen. Das Dielektrikum 206 kann auch ein Fasermaterial 208 aufweisen, das nicht Teil des Verbundwerkstoffs 26 ist.

Ferner umfasst die Sensoreinrichtung 200 eine Außenelektrode 210, die porös ist und die im Querschnitt betrachtet die erste Innenelektrode 202 und die zweite Innenelektrode 204 vollständig umgibt. Die Außenelektrode 210 begrenzt einen Probenraum 212, in dem das Dielektrikum 206 oder der Verbundwerkstoff 26 angeordnet sein kann.

Die Außenelektrode 210 ist mit Öffnungen oder Poren derart versehen, dass ein Matrixmaterial beim Infusionieren des Verbundwerkstoffes durch die Außenelektrode 210 hindurchtreten und in den Probenraum 212 eindringen kann.

Vorzugsweise werden bei bestimmungsgemäßer Verwendung die erste Innenelektrode 202 und die zweite Innenelektrode 204 mit Spannungsimpulsen beaufschlagt, wohingegen die Außenelektrode 210 an Erde angeschlossen ist.

Nachfolgend wird anhand Fig. 6 ein weiteres Ausführungsbeispiel einer Sensoreinrichtung 78 beschrieben. Die Sensoreinrichtung 78 erstreckt sich in einer axialen Richtung.

Die Sensoreinrichtung 78 weist eine Innenelektrode 80 (Beispiel für eine erste Elektrode) auf. Die Innenelektrode 80 ist beispielsweise als Zylinderstab 82 ausgebildet. Der Zylinderstab 82 fungiert als ein Flechtkern 84. Die Innenelektrode 80 ist aus einem leitenden Material, vorzugsweise Stahl, hergestellt.

Die Sensoreinrichtung 78 umfasst ferner eine Außenelektrode 86 (Beispiel für eine zweite Elektrode). Die Außenelektrode 86 umgibt im Querschnitt betrachtet die Innenelektrode 80 vollständig. Ferner ist die Außenelektrode 86 als Zylinderring 88 ausgebildet. Die Außenelektrode 86 ist vorzugsweise konzentrisch zu der Innenelektrode 80 angeordnet.

Die Sensoreinrichtung 78 weist einen Probenraum 90 auf, der zwischen der Innenelektrode 80 und der Außenelektrode 86 angeordnet und durch diese begrenzt ist. Der Probenraum 90 ist geeignet, den Verbundwerkstoff 26 aufzunehmen.

Die Sensoreinrichtung 78 umfasst zudem eine Vorschubeinheit 92. Die Vorschubeinheit 92 ist ausgebildet, den Verbundwerkstoff 26 in axialer Richtung relativ zu der Innenelektrode 80 und/oder der Außenelektrode 86 durch den Probenraum 90 zu bewegen.

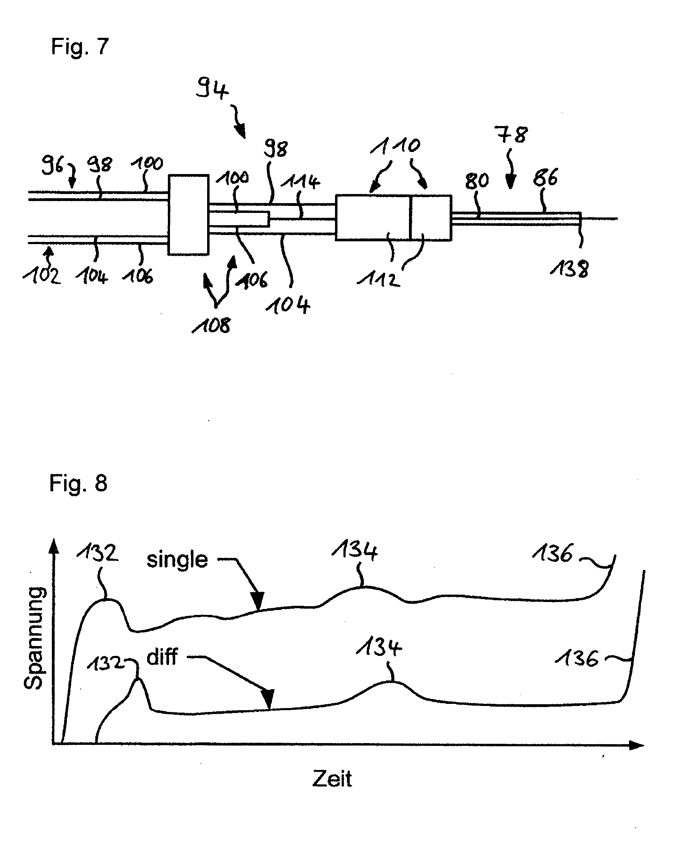

Anhand Fig. 7 wird nachfolgend eine Verbindungsanordnung 94 für Sensoreinrichtungen, wie beispielsweise die Sensoreinrichtung 78, beschrieben.

Die Verbindungsanordnung 94 umfasst ein erstes Koaxialkabel 96 mit einem ersten Innenleiter 98 und einem ersten Außenleiter 100. Ferner umfasst die Verbindungsanordnung 94 ein zweites Koaxialkabel 102 mit einem zweiten Innenleiter 104 und einem zweiten Außenleiter 106.

Die Verbindungsanordnung 94 umfasst eine erste Verbindungseinrichtung 108 zum Verbinden des ersten Koaxialkabels 96 mit dem zweiten Koaxialkabel 102. Ferner umfasst die Verbindungsanordnung 94 eine zweite Verbindungseinrichtung 110 zum Verbinden des ersten Koaxialkabels 96 und des zweiten Koaxialkabels 102 mit der Sensoreinrichtung 78. Die zweite Verbindungseinrichtung 110 weist vorzugsweise einen Steckverbinder 112 auf.

Nachfolgend wird die Verschaltung der Verbindungsanordnung 94 erläutert. Die erste Verbindungseinrichtung 108 verbindet den ersten Außenleiter 100 mit dem zweiten Außenleiter 106, so dass das erste Koaxialkabel 96 und das zweite Koaxialkabel 102 einen gemeinsamen Erdleiter 114 aufweisen. Der erste Innenleiter 98 und der zweite Innenleiter 104 werden durch den gemeinsamen Erdleiter 114 abgeschirmt. Die zweite Verbindungseinrichtung 110 wird ebenfalls durch den gemeinsamen Erdleiter 114 geschirmt. Der erste Innenleiter 98 ist beispielsweise mit der Innenelektrode 80 verbunden und der zweite Innenleiter 104 ist mit der Außenelektrode 86 verbunden.

Somit kann eine Abschirmung der Zuleitung zu der Sensoreinrichtung 78 bis zu dem Beginn der Sensoreinrichtung 78 erreicht werden. Der Einfluss von äußeren Störsignalen ist dadurch verringert.

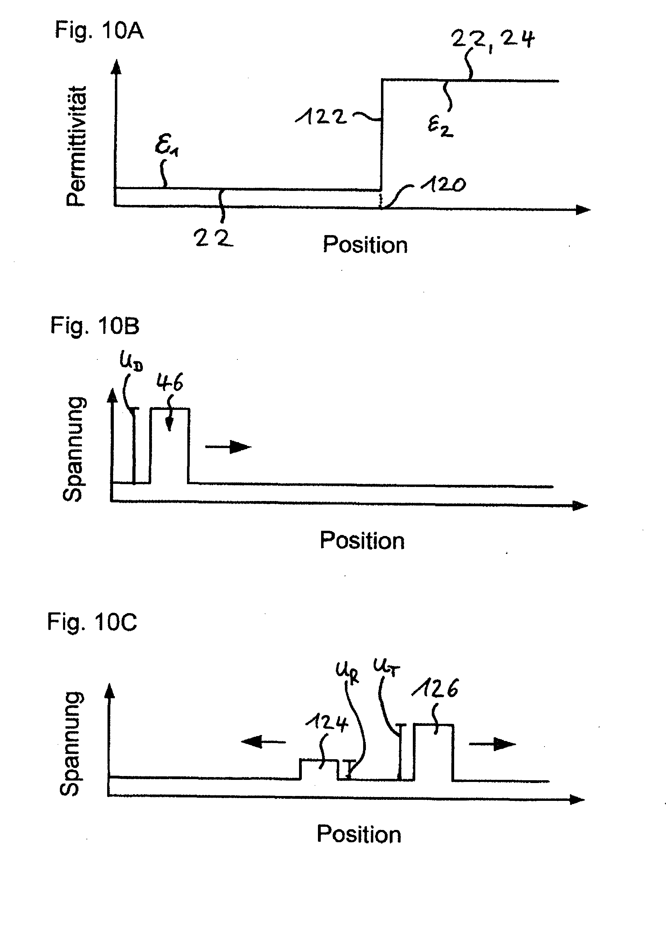

Es wird nachfolgend auf die Fig. 1, 8, 10A bis 10C Bezug genommen, anhand derer ein Ausführungsbeispiel eines Materialparameterermittlungsverfahrens beschrieben wird.

Fig. 8 zeigt schematisch ein Messergebnis einer single-ended TDR-Messung und einer differentiellen TDR-Messung. Fig. 8A zeigt schematisch den räumlichen Verlauf der dielektrischen Permittivität des Verbundwerkstoffes 26. Der Verbundwerkstoff 26 weist beispielsweise eine erste Permittivität $\varepsilon_1$ und eine zweite Permittivität $\varepsilon_2$ auf. An einer Sprungstelle 120 ist ein Permittivitätsübergang 122 zwischen der ersten Permittivität $\varepsilon_1$ und der zweiten Permittivität $\varepsilon_2$ gebildet. Der Permittivitätsübergang 122 entsteht beispielsweise durch den Übergang zwischen einem Bereich des Fasermaterial 22 ohne das Matrixmaterial 24 und einem Bereich des Fasermaterials 22 mit dem Matrixmaterial 24 oder auch Fehlern in dem Fasermaterial 22 oder in dem Matrixmaterial 24.

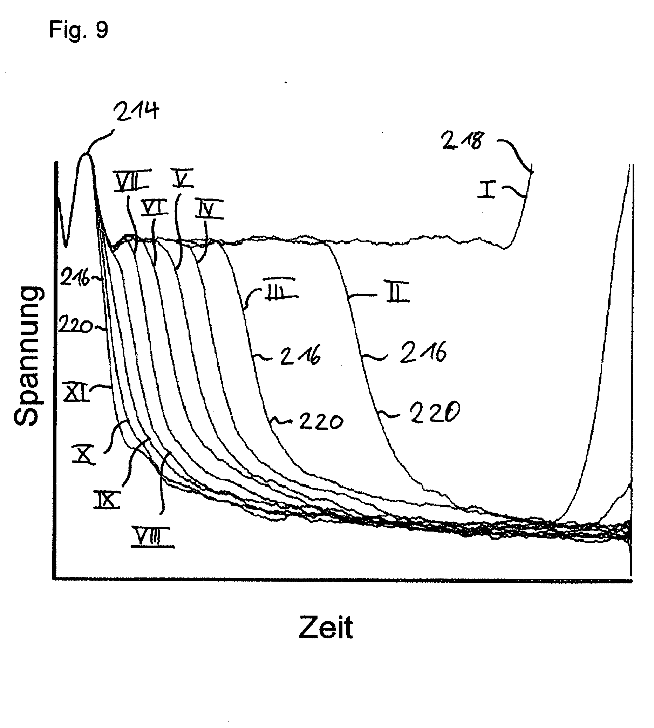

Fig. 9B zeigt schematisch den räumlichen Spannungsverlauf entlang der axialen Richtung einer Sensoreinrichtung, wie beispielsweise der Sensoreinrichtung 78. Schematisch dargestellt ist der Detektionsspannungsimpuls 46, der sich in Pfeilrichtung entlang der Innenelektrode 80 bewegt. Trifft der Detektionsspannungsimpuls 46 an der Sprungstelle 120 auf den Permittivitätsübergang 122, entstehen ein Reflexionsspannungsimpuls 124 mit einer Reflexionsspannungsimpulsamplitude $U_R$ und ein Transmissionsspannungsimpuls 126 mit einer Transmissionsspannungsimpulsamplitude $U_T$. Es gilt im Wesentlichen:

$$U_R \propto \frac{\sqrt{\varepsilon_1} - \sqrt{\varepsilon_2}}{\sqrt{\varepsilon_1} + \sqrt{\varepsilon_2}}$$

Der Reflexionsspannungsimpuls 124 läuft von der Sprungstelle 120 zurück zu der Materialparameterermittlungsvorrichtung 20 und wird dort von dem Spannungsimpulserfassungsmodul 48 erfasst. Insbesondere wird die Reflexionsspannungsimpulsamplitude $U_R$ von dem Spannungsimpulserfassungsmodul 48 erfasst. Beim Aussenden des Detektionsspannungsimpulses 46 wird von dem Spannungsimpulserzeugungsmodul 44 ein Startsignal 128 an das Spannungsimpulslaufzeiterfassungsmodul 50 gesendet. Beim Empfangen des Reflexionsspannungsimpulses 124 sendet das Spannungsimpulserfassungsmodul 48 ein Stoppsignal 130 an das Spannungsimpulslaufzeiterfassungsmodul 50. Das Spannungsimpulslaufzeiterfassungsmodul 50 ermittelt den Zeitabstand zwischen dem Startsignal 128 und dem Stoppsignal 130 und daraus eine Spannungsimpulslaufzeit t. Die Spannungsimpulslaufzeit t und die Reflexionsspannungsimpulsamplitude $U_R$ werden an das Auswertemodul 52 übermittelt. Das Auswertemodul 52 bestimmt aus der Spannungsimpulslaufzeit t und dem Verhältnis aus Reflexionsspannungsimpulsamplitude $U_R$ und Detektionsspannungsimpulsamplitude $U_D$ das Verhältnis von erster Permittivität $\varepsilon_1$ und zweiter Permittivität $\varepsilon_2$ sowie die Position der Sprungstelle 120 in der Zeitdomäne.

Da die dielektrischen Eigenschaften des Fasermaterials 22 und des Matrixmaterials 24 gut bekannt sind, bestimmt das Auswertemodul 52 daraus die räumliche Position der Sprungstelle 120 entlang der Sensoreinrichtung 78 und die Höhe des Permittivitätsübergangs 122, d.h. die Änderung zwischen erster Permittivität $\varepsilon_1$ und zweiter Permittivität $\varepsilon_2$.

Als Ergebnis erhält man beispielsweise die in der Fig. 8 mit "single" bezeichnete Messkurve. Es sind im Wesentlichen eine erste Sprungstelle 132, eine zweite Sprungstelle 134 und eine dritte Sprungstelle 136 zu erkennen. Die erste Sprungstelle 132 kann auf den Anschluss der Sensoreinrichtung 78 zurückgeführt werden. Die zweite Sprungstelle 134 gibt beispielsweise die momentane Position der Fließfront des Matrixmaterials 22 an. Aus dem Amplitudenverhältnis des Reflexionsspannungsimpulsamplitude $U_R$ und der Detektionsspannungsimpulsamplitude $U_D$ lässt sich der Aushärtungsgrad des Matrixmaterials 24 ableiten. Die dritte Sprungstelle 136 ist stark ausgeprägt und kann auf einen Endabschnitt 138 der Sensoreinrichtung 78 zurückgeführt werden. Der Endabschnitt 138 weist aufgrund des abrupten Übergangs zwischen der Sensoreinrichtung 78 und Luft eine hohe Reflektivität auf, so dass die Reflexionsspannungsimpulsamplitude $U_R$ entsprechend groß ist.

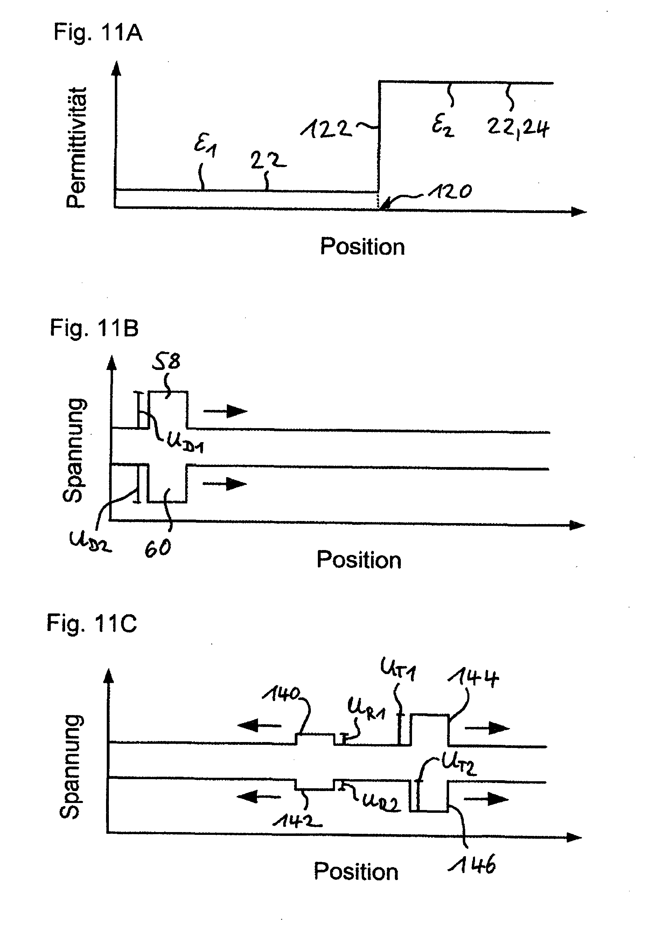

Es wird nachfolgend auf die Fig. 2, 8, 9, 11A bis 11C Bezug genommen, anhand derer ein Ausführungsbeispiel eines Materialparameterermittlungsverfahrens beschrieben wird.

Fig. 11A zeigt schematisch die gleiche Darstellung wie Fig. 10A, so dass diese nicht näher beschrieben wird.

Fig. 11B zeigt schematisch den jeweils von der Materialparameterermittlungsvorrichtung 54 erzeugten ersten Detektionsspannungsimpuls 58 und den zweiten Detektionsspannungsimpuls 60. Wie aus Fig. 9B ersichtlich, haben der erste Detektionsspannungsimpuls 58 und der zweite Detektionsspannungsimpuls eine gegengleiche Spannungs-

impulsamplitude. Es gilt:

$$U_{D1} = -U_{D2}$$

Der erste Detektionsspannungsimpuls 58 und der zweite Detektionsspannungsimpuls 60 bewegen sich entlang der axialen Richtung einer Sensoreinrichtung, wie etwa der Sensoreinrichtung 78, jeweils in der Innenelektrode 80 und der Außenelektrode 86.

Treffen der erste Detektionsspannungsimpuls 58 und der zweite Detektionsspannungsimpuls 60 auf den Permittivitätsübergang 122 an der Sprungstelle 120, so entstehen jeweils ein erster Reflexionsspannungsimpuls 140 mit einer ersten Reflexionsspannungsimpulsamplitude $U_{R1}$ und ein zweiter Reflexionsspannungsimpuls 142 mit einer zweiten Reflexionsspannungsimpulsamplitude $U_{R2}$. Ebenso entstehen jeweils ein erster Transmissionsspannungsimpuls 144 mit einer ersten Transmissionsspannungsimpulsamplitude $U_{T1}$ und ein zweiter Transmissionsspannungsimpuls 146 mit einer zweiten Transmissionsspannungsimpulsamplitude $U_{T2}$. Es gilt im Wesentlichen:

$$U_{Ri} \propto \frac{\sqrt{\varepsilon_1} - \sqrt{\varepsilon_2}}{\sqrt{\varepsilon_1} + \sqrt{\varepsilon_2}}; i = 1, 2$$

Beim Aussenden des ersten Detektionsspannungsimpulses 58 und des zweiten Detektionsspannungsimpulses 60 sendet das Spannungsimpulserzeugungsmodul 56 ein Startsignal 148 an das Spannungsimpulslaufzeiterfassungsmodul 50. Beim Empfangen des ersten Reflexionsspannungsimpulses 140 und des zweiten Reflexionsspannungsimpulses 142 sendet das Spannungsimpulserfassungsmodul 62 ein Stoppsignal 150 an das Spannungsimpulslaufzeiterfassungsmodul 50. Ferner ermittelt das Spannungsimpulserfassungsmodul 62 eine Gesamtreflexionsspannungsimpulsamplitude $U_G$ aus der ersten Reflexionsspannungsimpulsamplitude $U_{R1}$ und der zweiten Reflexionsspannungsimpulsamplitude $U_{R2}$. Es gilt:

$$U_G = U_{R1} - U_{R2}$$

Die Gesamtreflexionsspannungsimpulsamplitude $U_G$ wird an das Auswertemodul 52 geleitet und ebenso wie die Reflexionsspannungsimpulsamplitude $U_R$ des vorigen Beispiels verarbeitet.

In Fig. 8 ist das Messergebnis dieser sogenannten differentiellen TDR-Messung mit "diff" bezeichnet. Durch die Nutzung differentieller TDR zur Überwachung der Fließfront und des Aushärtegrades kann der Einfluss leitender Materialien in der Umgebung der Sensoreinrichtung 78 weiter reduziert und die Genauigkeit solcher Messungen in derartigen Umgebungen gegenüber der single-ended Messung weiter verbessert werden. Dies ist unter anderem darauf zurückzuführen, dass der erste Detektionsspannungsimpuls 58 und der zweite Detektionsspannungsimpuls 60 von äußeren Einflüssen gleichermaßen betroffen sind, so dass diese bei der Differenzbildung eliminiert werden.

In Fig. 9 ist beispielhaft ein Messergebnis einer differentiellen TDR-Messung dargestellt, wie es beispielsweise durch die Messung mit der Sensoreinrichtung 200 erhalten wird. In Fig. 9 ist die erfasste Spannung gegenüber der Zeit aufgetragen.

Die Messkurven werden mit I bis XI bezeichnet. Die Messkurve I ist im "trockenen" Zustand der Sensoreinrichtung 200 aufgenommen. In diesem Zustand befindet sich lediglich das Dielektrikum 206 in dem Probenraum 212. Das Fasermaterial 22 ist außerhalb der Außenelektrode 210 vorgesehen.

In Messkurve I sind eine erste Sprungstelle 214 und eine dritte Sprungstelle 218 erkennbar. Die erste Sprungstelle 214 rührt von dem Übergang in die Sensoreinrichtung 200 her. Die dritte Sprungstelle 218 ist auf einen Endabschnitt der Sensoreinrichtung 200 zurückzuführen.

Sodann wird die Infusion des Fasermaterials 22 begonnen. Das Matrixmaterial 24 breitet sich in dem Fasermaterial 22 aus. Trifft das Matrixmaterial 24 auf die Sensoreinrichtung 200, dringt das Matrixmaterial 24 durch die poröse Außenelektrode 210 in den Probenraum 212 und damit das Dielektrikum 206 ein. Eine Fließfront des Matrixmaterials 24 erzeugt einen Permittivitätsübergang 220 bei einer zweiten Sprungstelle 220. Es entsteht eine Messkurve ähnlich der Kurve II.

Wie aus der Messkurve II ersichtlich ist befindet sich der Permittivitätsübergang 220, d. h. die Fließfront des Matrixmaterials 24, etwa auf halber Länge der Sensoreinrichtung 200. Mit zunehmender Infusionszeit dringt mehr Matrixmaterial 24 in die Sensoreinrichtung 200 ein. Die Fließfront des Matrixmaterials 24 wandert immer weiter entlang der Sensoreinrichtung 200. Der Permittivitätsübergang 220 wandert somit weiter in Richtung der ersten Sprungstelle 214 (Messkurve III bis X). Schließlich wird ein stabiler Endzustand erreicht, wie er durch die Messkurve XI dargestellt ist. In diesem Zustand hat das Matrixmaterial 24 die Sensoreinrichtung 200 nahezu vollständig ausgefüllt.

Die Materialparameterermittlungsvorrichtungen 20, 54 können in einer Variante auch auf die Stoppsignale 130, 150 verzichten. Stattdessen wird dann lediglich das Startsignal 128, 148 an das Spannungsimpulslaufzeiterfassungsmodul 50 gesendet. Ferner wird kontinuierlich die momentan an dem Spannungsimpulserfassungsmodul 48, 62 anliegende Spannung an das Spannungsimpulslaufzeiterfassungsmodul 50 übermittelt und mit einem Zeitstempel versehen. Der Zeitstempel umfasst die seit dem erhalten des Startsignales 128, 148 verstrichene Zeit. Die Wertepaare Zeitstempel und Spannung werden an das Auswertemodul 52 zum Auswerten übermittelt. Es ist auch denkbar, dass die seit dem Erhalten des Startsignales 128, 148 verstrichene Zeit unabhängig von der momentan anliegenden Spannung an das Auswertemodul 52 übertragen wird.

Die hierin beschriebenen Vorrichtungen und Verfahren können auch mit bekannten Sensoreinrichtungen aus dem Stand der Technik, wie beispielsweise der Zweidrahtleitung aus Fig. 12 kombiniert werden. Mit den beschriebenen Vorrichtungen und Verfahren lässt sich der Herstellungsprozess des Verbundwerkstoffes 26 überwachen. Insbesondere können Fehler in dem Fasermaterial 22 oder beim Infusionieren und Aushärten des Matrixmaterials 24 erfasst werden. Ferner kann beim Aushärten des Matrixmaterials 24 der Aushärtegrad ermittelt werden. Ebenso kann die Position oder die Verteilung der Fließfront 220 des Matrixmaterials 24 in dem Fasermaterial 22 oder dem Verbundwerkstoff 26 bestimmt werden. Damit lässt sich der Herstellungsprozess des Verbundwerkstoffes 26 umfangreicher und genauer als bisher überwachen und steuern. Die Herstellung des Verbundwerkstoffes kann so weiter automatisiert und somit weniger aufwändig gestaltet werden. Verbleiben die Sensoreinrichtungen 28, 64, 78, 200 nach dem Aushärten in dem Verbundwerkstoff 26, kann eine Überwachung des Verbundwerkstoffes 26 auch im verbauten Zustand durchgeführt werden. Beschädigungen bei der Nutzung und Alterungsprozesse können somit ebenfalls nachvollzogen werden.

**Bezugszeichenliste:**

|  |  |
|---|---|
| 10 | erste Leitung |
| 12 | zweite Leitung |
| 14 | Dielektrikum |
| 16 | Fasermaterial |
| 18 | Harz |
| 20 | Materialparameterermittlungsvorrichtung |
| 22 | Fasermaterial |
| 24 | Matrixmaterial |
| 26 | Verbundwerkstoff |
| 28 | Sensoreinrichtung |
| 30 | Innenelektrode |
| 32 | Draht |
| 34 | Außenelektrode |
| 36 | Kupfergeflecht |
| 38 | Probenraum |
| 40 | Dielektrikum |
| 42 | Steuereinrichtung |
| 44 | Spannungsimpulserzeugungsmodul |
| 46 | Detektionsspannungsimpuls |
| 48 | Spannungsimpulserfassungsmodul |
| 50 | Spannungsimpulslaufzeiterfassungsmodul |
| 52 | Auswertemodul |
| 54 | Materialparameterermittlungsvorrichtung |
| 56 | Spannungsimpulserzeugungsmodul |
| 58 | erster Detektionsspannungsimpuls |

EP 3 035 042 A1

| 60 | zweiter Detektionsspannungsimpuls |
| 62 | Spannungsimpulserfassungsmodul |
| 64 | Sensoreinrichtung |
| 66 | Innenelektrode |
| 68 | Litze |
| 70 | Außenelektrode |
| 72 | Geflecht |
| 74 | Probenraum |
| 76 | Isolierhülle |
| 78 | Sensoreinrichtung |
| 80 | Innenelektrode |
| 82 | Zylinderstab |
| 84 | Flechtkern |
| 86 | Außenelektrode |
| 88 | Zylinderring |
| 90 | Probenraum |
| 92 | Vorschubeinheit |
| 94 | Verbindungsanordnung |
| 96 | erstes Koaxialkabel |
| 98 | erster Innenleiter |
| 100 | erster Außenleiter |
| 102 | zweites Koaxialkabel |
| 104 | zweiter Innenleiter |
| 106 | zweiter Außenleiter |
| 108 | erste Verbindungseinrichtung |
| 110 | zweite Verbindungseinrichtung |
| 112 | Steckverbinder |
| 114 | Erdleiter |
| 120 | Sprungstelle |
| 122 | Permittivitätsübergang |
| 124 | Reflexionsspannungsimpuls |
| 126 | Transmissionsspannungsimpuls |
| 128 | Startsignal |
| 130 | Stoppsignal |
| 132 | erste Sprungstelle |
| 134 | zweite Sprungstelle |
| 136 | dritte Sprungstelle |
| 138 | Endabschnitt |
| 140 | erster Reflexionsspannungsimpuls |
| 142 | zweiter Reflexionsspannungsimpuls |
| 144 | erster Transmissionsspannungsimpuls |
| 146 | zweiter Transmissionsspannungsimpuls |
| 148 | Startsignal |
| 150 | Stoppsignal |
| 200 | Sensoreinrichtung |
| 202 | erste Innenelektrode |
| 204 | zweite Innenelektrode |
| 206 | Dielektrikum |
| 208 | Fasermaterial |
| 210 | Außenelektrode |
| 212 | Probenraum |
| 214 | erste Sprungstelle |
| 216 | zweite Sprungstelle |
| 218 | dritte Sprungstelle |
| 220 | Permittivitätsübergang |
| $\varepsilon_1$ | erste Permittivität |
| $\varepsilon_2$ | zweite Permittivität |

t        Spannungsimpulslaufzeit
$U_D$       Detektionsspannungsimpulsamplitude
$U_{D1}$      erste Detektionsspannungsimpulsamplitude
$U_{D2}$      zweite Detektionsspannungsimpulsamplitude
$U_G$       Gesamtreflexionsspannungsimpulsamplitude
$U_R$       Reflexionsspannungsimpulsamplitude
$U_{R1}$      erste Reflexionsspannungsimpulsamplitude
$U_{R2}$      zweite Reflexionsspannungsimpulsamplitude
$U_T$       Transmissionsspannungsimpulsamplitude
$U_{T1}$      erste Transmissionsspannungsimpulsamplitude
$U_{T2}$      zweite Transmissionsspannungsimpulsamplitude

**Patentansprüche**

1. Sensoreinrichtung (28, 64, 78, 200) zum Erfassen wenigstens eines Materialparameters eines Verbundwerkstoffs (26), der ein Matrixmaterial (24) aufweist, um Messdaten zu erhalten, mit einem zwischen einer Innenelektrode (30, 66, 80, 202, 204) und einer die Innenelektrode (30, 66, 80, 202, 204) im Querschnitt vollständig umgebenden Außenelektrode (34, 70, 86, 210) vorgesehenen Probenraum (38, 74, 90, 212) zum Aufnehmen des Matrixmaterials (24) und/oder des Verbundwerkstoffs (26).

2. Sensoreinrichtung (200) nach einem der vorstehenden Ansprüche, **gekennzeichnet durch** eine zweite Innenelektrode (204), die in dem Probenraum (212) angeordnet ist.

3. Sensoreinrichtung (28, 64, 78, 200) nach einem der vorstehenden Ansprüche, **dadurch gekennzeichnet, dass** die Innenelektrode (30, 66, 80, 202) und/oder die zweite Innenelektrode (204)

   3.1 einen Draht (32) und/oder
   3.2 eine Litze (68) und/oder
   3.3 einen Flechtkern (84) und/oder
   3.4 einen nach dem Aushärten des Verbundwerkstoffs (26) aus diesem entfernbaren Abschnitt und/oder
   3.5 einen nach dem Aushärten des Verbundwerkstoffs (26) aus diesem nicht-entfernbaren Abschnitt aufweist.

4. Sensoreinrichtung (28, 64, 78, 200) nach einem der vorstehenden Ansprüche, **dadurch gekennzeichnet, dass** die Außenelektrode (34, 70, 86, 210)

   4.1 im Querschnitt ringförmig und/oder kreisringförmig ausgebildet ist und/oder
   4.2 im Querschnitt rechteckig und/oder quadratisch ausgebildet ist und/oder
   4.3 abschnittsweise oder vollständig konzentrisch zu der Innenelektrode (30, 66, 80) angeordnet ist und/oder
   4.4 ein poröses Material und/oder
   4.5 Öffnungen und/oder
   4.6 Poren und/oder
   4.7 ein leitendes Material und/oder
   4.8 ein Geflecht (72) und/oder
   4.9 ein Gelege und/oder
   4.10 eine Konsolidierungseinrichtung zum Aushärten des Verbundwerkstoffs (26) aufweist.

5. Sensoreinrichtung (28, 64, 78, 200) nach einem der vorstehenden Ansprüche, **dadurch gekennzeichnet, dass** die Innenelektrode (30, 66, 80) und/oder die zweite Innenelektrode (202, 204) und/oder die Außenelektrode (34, 70, 86, 210) aus Metall und/oder Kupfer und/oder Stahl gefertigt sind.

6. Sensoreinrichtung (28, 64, 78, 200) nach einem der vorstehenden Ansprüche, **dadurch gekennzeichnet, dass** der Probenraum (38, 74, 90, 212) zum Aufnehmen eines Fasermaterials (22) und/oder des Matrixmaterials (24) und/oder des Verbundwerkstoffs (26) ausgebildet ist.

7. Messanordnung **gekennzeichnet durch** eine Sensoreinrichtung (28, 64, 78, 200) nach einem der vorstehenden Ansprüche, wobei der Probenraum (38, 74, 90, 212) ein Dielektrikum (40, 206) aufweist,

6.1 das teilweise oder vollständig aus einem Fasermaterial (22) und/oder einem Matrixmaterial (24) und/oder dem Verbundwerkstoff (26) gebildet ist und/oder

6.2 das in der axialen Richtung der Sensoreinrichtung (28, 64, 78, 200) bewegbar ist.

**8.** Materialparametererfassungsverfahren zum Erfassen wenigstens eines Materialparameters eines Verbundwerkstoffs (26), **gekennzeichnet durch** die Schritte:

8.1 Anordnen des Verbundwerkstoffs (26) in einem zwischen einer Innenelektrode (30, 66, 80, 202, 204) und einer die Innenelektrode (30, 66, 80, 202, 204) im Querschnitt vollständig umgebenden Außenelektrode (34, 70, 86, 210) vorgesehenen Probenraum (38, 74, 90, 212) zum Aufnehmen des Matrixmaterials (24) und/oder des Verbundwerkstoffs (26) ausgebildet ist;

8.2 Einspeisen eines Detektionsspannungsimpulses (46, 58, 60) mit einer Detektionsspannungsimpulsamplitude ($U_D$, $U_{D1}$, $U_{D2}$) in die Innenelektrode (30, 66, 80, 202);

8.3 Erfassen einer Reflexionsspannungsimpulslaufzeit (t) und/oder einer Reflexionsspannungsimpulsamplitude ($U_R$, $U_{R1}$, $U_{R2}$, $U_G$) eines aus dem Detektionsspannungsimpuls (46, 58, 60) hervorgegangenen Reflexionsspannungsimputses (124, 140, 142);

8.4 Ermitteln des Materialparameters aus der Reflexionsspannungsimpulslaufzeit (t) und/oder der Reflexionsspannungsimpulsamplitude ($U_R$, $U_{R1}$, $U_{R2}$, $U_G$).

**9.** Verfahren nach Anspruch 8, **gekennzeichnet durch** den Schritt:

9.1 Anschließen der Außenelektrode (34, 70, 86, 210) an Erde.

**10.** Verfahren nach Anspruch 8 oder 9, **dadurch gekennzeichnet, dass** Schritt 8.1 einen der folgenden Schritte enthält:

10.1 Bewegen des Verbundwerkstoffs (26) durch den Probenraum (38, 74, 90, 212); und/oder

10.2 Bewegen des Verbundwerkstoffs (26) durch den Probenraum (38, 74, 90, 212) entlang der axialen Richtung des Probenraums (38, 74, 90, 212); und/oder

10.3 Relativbewegen des Verbundwerkstoffs (26) relativ zu der Innenelektrode (30, 66, 80, 202, 204) und/oder zu der Außenelektrode (212); und/oder

10.4 Relativbewegen des Verbundwerkstoffs (26) relativ zu der Innenelektrode (30, 66, 80, 202, 204) und/oder zu der Außenelektrode (212) entlang der axialen Richtung.

**11.** Verfahren nach einem der Ansprüche 8 bis 10, **gekennzeichnet durch** Verwenden einer Sensoreinrichtung (28, 64, 78, 200) nach einem der Ansprüche 1 bis 6 oder Verwenden einer Messanordnung nach Anspruch 7.

Fig. 1

Fig. 2

Fig. 3

Fig. 4

Fig. 5

Fig. 6

Fig. 7

Fig. 8

Fig. 9

Fig. 10A

Fig. 10B

Fig. 10C

Fig. 11A

Fig. 11B

Fig. 11C

Fig. 12

Stand der Technik

Europäisches
Patentamt

European
Patent Office

Office européen
des brevets

**EUROPÄISCHER RECHERCHENBERICHT**

Nummer der Anmeldung

EP 14 00 4272

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | Hugo Fellner-Feldegg: "Measurement of dielectrics in the time domain", The Journal of Physical Chemistry, 1. März 1969 (1969-03-01), Seiten 616-623, XP055192808, DOI: 10.1021/j100723a023 Gefunden im Internet: URL:http://pubs.acs.org/doi/pdf/10.1021/j100723a023 * Seiten 616,617 * * Seiten 619,620 * | 1-11 | INV. G01N27/22 |
| X | JP S59 216047 A (YAMAMOTO ELECTRIC IND CO LTD) 6. Dezember 1984 (1984-12-06) * Zusammenfassung; Abbildungen 3-5 * | 1-7 | |
| X | US 4 786 857 A (MOHR CHARLES L [US] ET AL) 22. November 1988 (1988-11-22) * das ganze Dokument * | 1-7 | |
| X | US 2004/027137 A1 (SHERRARD WAYNE [CA]) 12. Februar 2004 (2004-02-12) * das ganze Dokument * | 1-7 | RECHERCHIERTE SACHGEBIETE (IPC) G01N |
| X | DE 195 11 556 C1 (DAIMLER BENZ AG [DE]) 25. Juli 1996 (1996-07-25) * das ganze Dokument * | 1,4-7 | |
| A | SCOTT B. JONES ET AL: "Time domain reflectometry measurement principles and applications", HYDROLOGICAL PROCESSES, Bd. 16, Nr. 1, 1. Januar 2002 (2002-01-01), Seiten 141-153, XP055192594, ISSN: 0885-6087, DOI: 10.1002/hyp.513 * das ganze Dokument * | 1-11 | |

-/--

Der vorliegende Recherchenbericht wurde für alle Patentansprüche erstellt

| Recherchenort | Abschlußdatum der Recherche | Prüfer |
|---|---|---|
| München | 2. Juni 2015 | Stussi, Elisa |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument

EPO FORM 1503 03.82 (P04C03)

Europäisches Patentamt
European Patent Office
Office européen des brevets

**EUROPÄISCHER RECHERCHENBERICHT**

Nummer der Anmeldung

EP 14 00 4272

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | AGNIESZKA SZYPLOWSKA ET AL: "Application of a Coaxial-Like Sensor for Impedance Spectroscopy Measurements of Selected Low-Conductivity Liquids", SENSORS, Bd. 13, Nr. 10, 30. September 2013 (2013-09-30), Seiten 13301-13317, XP055192641, DOI: 10.3390/s131013301 * das ganze Dokument * ----- | 1-11 | |
| A,D | DOMINAUSKAS A ET AL: "Electric time-domain reflectometry sensor for online flow sensing in liquid composite molding processing", COMPOSITES PART A: APPLIED SCIENCE AND MANUFACTURING, ELSEVIER SCIENCE PUBLISHERS B.V., AMSTERDAM, NL, Bd. 34, Nr. 1, 1. Januar 2003 (2003-01-01), Seiten 67-74, XP004410247, ISSN: 1359-835X, DOI: 10.1016/S1359-835X(02)00232-4 * das ganze Dokument * ----- | 1-11 | |
| A,D | DOMINAUSKAS ET AL: "Electric time-domain reflectometry distributed flow sensor", COMPOSITES PART A: APPLIED SCIENCE AND MANUFACTURING, ELSEVIER SCIENCE PUBLISHERS B.V., AMSTERDAM, NL, Bd. 38, Nr. 1, 25. November 2006 (2006-11-25), Seiten 138-146, XP005780513, ISSN: 1359-835X, DOI: 10.1016/J.COMPOSITESA.2006.01.019 * das ganze Dokument * ----- | 1-11 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 2. Juni 2015 | Stussi, Elisa |

**EP 3 035 042 A1**

**ANHANG ZUM EUROPÄISCHEN RECHERCHENBERICHT**
**ÜBER DIE EUROPÄISCHE PATENTANMELDUNG NR.**

EP 14 00 4272

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-06-2015

| Im Recherchenbericht angeführtes Patentdokument | Datum der Veröffentlichung | Mitglied(er) der Patentfamilie | Datum der Veröffentlichung |
|---|---|---|---|
| JP S59216047 A | 06-12-1984 | KEINE | |
| US 4786857 A | 22-11-1988 | DE 3788590 D1<br>DE 3788590 T2<br>EP 0244033 A2<br>US 4786857 A | 10-02-1994<br>14-07-1994<br>04-11-1987<br>22-11-1988 |
| US 2004027137 A1 | 12-02-2004 | CA 2384257 A1<br>US 2004027137 A1 | 29-10-2003<br>12-02-2004 |
| DE 19511556 C1 | 25-07-1996 | AR 001433 A1<br>BR 9607782 A<br>CN 1179828 A<br>DE 19511556 C1<br>EP 0817964 A1<br>JP H11507434 A<br>US 6250152 B1<br>WO 9630758 A1 | 22-10-1997<br>07-07-1998<br>22-04-1998<br>25-07-1996<br>14-01-1998<br>29-06-1999<br>26-06-2001<br>03-10-1996 |

EPO FORM P0461

Für nähere Einzelheiten zu diesem Anhang : siehe Amtsblatt des Europäischen Patentamts, Nr.12/82

24

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **SERNEK ; KAMKE.** *Int. J. Adhes. Adhes.,* 2007, vol. 27 (7), 562-567
- **KAZILAS ; PARTRIDGE.** *Poymer,* vol. 46 (16), 5868-5878
- **HARDIS et al.** *Compos. Part Appl. Sci. Manuf.,* 2013, vol. 49, 100-108
- **DOMINAUSKAS et al.** *Compos. Part Appl. Sci. Manuf.,* 2003, vol. 34, 67-74
- *COMPOS. PART APPL. SCI. MANUF.,* 2007, vol. 38, 138-146